# EUROPEAN PATENT SPECIFICATION

(11) **EP 4 106 772 B1**
(45) Date of publication and mention of the grant of the patent: **04.11.2026**
(21) Application number: 21745405.7
(22) Date of filing: 11.06.2021
(51) Int. Cl.: A61K 31/728, A61K 9/00, A61K 45/06, A61P 27/02

(54) **HIGH MOLECULAR WEIGHT HYALURONIC ACID FOR USE IN THE TREATMENT OF CORNEAL NERVE DAMAGE OR LOSS**
HYALURONSÄURE MIT HOHEM MOLEKULARGEWICHT ZUR VERWENDUNG BEI DER BEHANDLUNG VON SCHÄDEN ODER VERLUST DER HORNHAUTNERVEN
ACIDE HYALURONIQUE DE HAUT POIDS MOLÉCULAIRE DESTINÉ À ÊTRE UTILISÉ DANS LE TRAITEMENT D'UNE LÉSION OU D'UNE PERTE DE NERFS CORNÉENS

(30) Priority: 12.06.2020 US 202063038361 P; 24.07.2020 US 202063056081 P
(43) Date of publication of application: 28.12.2022
(73) Proprietor: i.com Medical GmbH, 81241 München (DE)
(72) Inventor: MÜLLER-LIERHEIM, Wolfgang Georg, Konrad, 81243 München (DE); VAN SETTEN, Gysbert-Botho, 18233 Danderyd (SE)
(74) Representative: Gill Jennings & Every LLP
(86) International application number: PCT/IB2021/000389
(87) International publication number: WO 2021/250462

(56) References cited:
- WO-A1-2017/053339
- WO-A1-2018/069763
- TW-A- 201 945 010
- KRZOSKA ET AL: "Effectiveness of Comfort Shield MDS results of a Clinical Investigation at Beuth Hocjschule für Tecknik, berlin", INTERNET CITATION, 1 July 2016 (2016-07-01), XP002777748, Retrieved from the Internet <URL:https://www.icom-medical.de/upload/15563389-Krzoska-Wirksamkeit-von-Comfort-Shield-Beuth-Studie-GALIFA-AUGENBLICK-2016-EN.pdf> [retrieved on 19000101]
- TAKASHI KOJIMA ET AL: "The Effects of High Molecular Weight Hyaluronic Acid Eye Drop Application in Environmental Dry Eye Stress Model Mice", INTERNATIONAL JOURNAL OF MOLECULAR SCIENCES, vol. 21, no. 10, 15 May 2020 (2020-05-15), pages 3516, XP055769490, DOI: 10.3390/ijms21103516
- VAN SETTEN GYSBERT-BOTHO ET AL: "High Molecular Weight Hyaluronan Promotes Corneal Nerve Growth in Severe Dry Eyes", JOURNAL OF CLINICAL MEDICINE, vol. 9, no. 12, 24 November 2020 (2020-11-24), pages 3799, XP055847490, DOI: 10.3390/jcm9123799
- VAN SETTEN GYSBERT-BOTHO ET AL: "The HYLAN M Study: Efficacy of 0.15% High Molecular Weight Hyaluronan Fluid in the Treatment of Severe Dry Eye Disease in a Multicenter Randomized Trial", JOURNAL OF CLINICAL MEDICINE, vol. 9, no. 11, 2 November 2020 (2020-11-02), pages 3536, XP055847493, DOI: 10.3390/jcm9113536

## Description

### BACKGROUND OF THE INVENTION

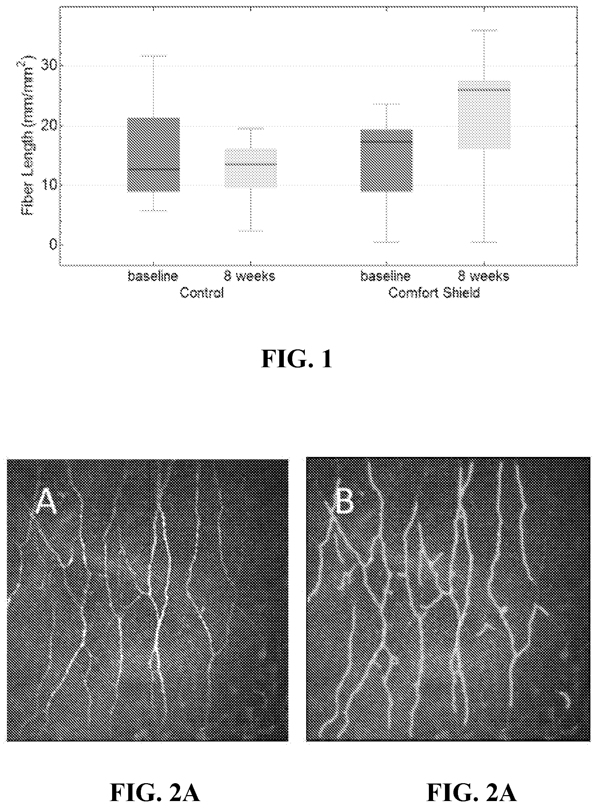

The cornea is a densely innervated surface tissue and has been studied using histochemical and *in vivo* confocal microscopy (IVCM) (see Guthoff RF et al., "Epithelial Innervation of Human Cornea - A Three-Dimensional Study Using Confocal Laser Scanning Fluorescence Microscopy", Cornea, 2005, 24(5): 608-613, particularly the schematic drawing of Figure 2 therein). In addition to their sensory function, corneal nerves contribute to blink reflex, tear production, and the maintenance of the ocular surface functional integrity through the release of trophic factors such as substance P (SP), calcitonin gene-related peptide (CGRP), epidermal growth factor (EGF), nerve growth factor (NGF), brain-derived neurotrophic factor (BDNF), and neurotrophin- (NT-) 3 (Shaheen, BS et al., "Corneal nerves in health and disease," Survey of Ophthalmology, 2014, vol. 59, pp. 263-285; Marfurt CF et al.. "Anatomy of the human corneal innervation," Exp Eye Res, 2010; 4:478-492; You L et al., "Neurotrophic factors in the human cornea," Invest Ophthalmol Vis Sci, 2000 Mar; 41(3):692-702).

Cell-cell interactions among different cell types play a significant role in maintaining nerve function and integrity, and these interactions are necessary for the repair of damaged tissue to restore a normal healthy state. Therefore, modulation of these cell-cell interactions is considered to be a potential regeneration strategy in nerve tissue, with the goal being to reestablish the functional epithelial and stromal microenvironment, including restoration of the corneal nerve interactions with surrounding cells, following a nerve injury. There is a great deal scientific literature on the origin (Kowtharapu BS and Stachs O, Corneal Cells: Fine-tuning Nerve Regeneration, Current Eye Research, 2020, 45(3):291-302).

Corneal nerve dysfunction due to mechanical or chemical trauma, inflammation, refractive surgery, infections, and other causative factors can result in corneal diseases. For example, because the corneal nerves play an important role in homeostasis of the corneal epithelium, neurotrophic keratopathy (NK) may develop when there is a disturbance of this homeostasis following damage or loss of nerves (Eguchi H et al., "Corneal Nerve Fiber Structure, Its Role in Corneal Function, and Its Changes in Corneal Diseases," Biomed Res Int., 2017, 2017: 3242649; Mastropasqua L et al., "Understanding the Pathogenesis of Neurotrophic Keratitis: The Role of Corneal Nerves, J. Cell. Physiol., 232: 717-724, 2017).

Progress has been made in the diagnosis of corneal nerve damage or loss using IVCM, which allows the direct visualization of the corneal sub-basal nerve plexus *in vivo* and the evaluation of the regeneration of the corneal nerves at different stages of disease or before and after a treatment, such as laser *in situ* keratomileusis (LASIK) (Choi EY et al., "Langerhans cells prevent sub-basal nerve damage and upregulate neurotrophic factors in dry eye disease", PLoS One, 2017; 12(4):e0176153); Tuisku, IS et al., "Alterations in corneal sensitivity and nerve morphology in patients with primary Sjogren's syndrome," Experimental Eye Research, 2008, vol. 86, pp. 879-885; Alhatem A et al., "In vivo confocal microscopy in dry eye disease and related conditions," Seminars in Ophthalmology, 2012, vol. 27, pp. 138-148; Patel DV and C. N. McGhee, "Quantitative analysis of in vivo confocal microscopy images: a review," Survey of Ophthalmology, 2013, vol. 58, pp. 466-475; Cruzat A. et al., "In vivo confocal microscopy of corneal nerves in health and disease," The Ocular Surface, vol. 15, pp. 15-47, 2017). Therapeutic strategies aimed at restoring the damaged or lost nerves are limited and expensive, however (John T et al., "Corneal Nerve Regeneration after Self-Retained Cryopreserved Amniotic Membrane in Dry Eye Disease," J Ophthalmol., 2017; 2017: 6404918).

WO2018/069763 relates to a method for reducing or delaying the onset of irritation, discomfort, inflammation (acute or chronic), and/or an immune response at the ocular surface by topically administering a fluid comprising high molecular weight hyaluronic acid to the ocular surface of the eye of a human or non-human animal subject, wherein the hyaluronic acid has an intrinsic viscosity of > 2.5 m³/kg and a concentration of < 0.2 % w/v.

Krzoska et al. (2016) ("Effectiveness of Comfort Shield MOS results of a Clinical Investigation at Beuth Hochschule für Technik, Berlin XP002777748) describes the effectiveness of Comfort Shield MDS in a clinical setting.

Kojima et al. (2020) (The Effects of High Molecular Weight Hyaluronic Acid Eye Drop Application in Environmental Dry Eye Stress Model Mice, Intern. J. Mol. Sci., 21(10): 3516) relates to a comparative study, which compares the effects of high and low molecular weight hyaluronic acid (HA) in the treatment of dry eye.

WO 2017/053339 relates to methods for alleviating pain and discomfort associated with a dry eye condition and methods for alleviating pain and discomfort while minimizing at least one skin imperfection or while facilitating wound healing.

TW 201 945 010 relates to the use of hyaluronic acid to treat nerve injuries.

Van Setten et al. (2020) (The HYLAN M Study: Efficacy of 0.15% High Molecular Weight Hyaluronan Fluid in the Treatment of Severe Dry Eye Disease in a Multicenter Randomized Trial," J Clin Med., 9(11):3536) relates to the HYLAN M study and describes the use of 0.15% high molecular weight hyaluronan fluid in the treatment of severe dry eye disease in a multicenter randomized trial.

### BRIEF SUMMARY OF THE INVENTION

The present invention relates to a fluid comprising high molecular weight hyaluronic acid (HMWHA) for use in treating or preventing corneal nerve damage or loss in a human or non-human animal subject, wherein the fluid is for topical administration to the ocular surface of the subject, wherein the hyaluronic acid has an intrinsic viscosity of at least 2.5 m3/kg, wherein the hyaluronic acid has a molecular weight in the range of 3 million to 4 million Daltons, and wherein the subject has diabetes and diabetic peripheral neuropathy, diabetes but does not yet have diabetic peripheral neuropathy, diabetic corneal neuropathy, stroke, neurotrophic keratopathy (NK), or corneal nerve damage or loss caused by trauma or a medical procedure. The corneal nerve damage or loss that may be treated using the method of the invention is any type of nerve injury that inhibits or impairs normal corneal nerve turnover, orientation, growth, function, or any combination of two or more of the foregoing.

### BRIEF DESCRIPTION OF THE DRAWINGS

The patent or application file contains at least one drawing executed in color. Copies of this patent or patent application publication with color drawing(s) will be provided by the Patent and Trademark Office upon request and payment of the necessary fee.
**Figure 1**. Total nerve fiber length is greater in patients treated with high molecular weight hyaluronic acid eye drops (COMFORT SHIELD^{®} preservative-free sodium hyaluronate eye drops) at 8 weeks compared to control lubricant eye drops.
**Figures 2A and 2B****.** Single image from the subbasal nerve plexus (SNP) in an individual (Figure 2A) and automatically detected nerve fibers used for quantification (Figure 2B).
**Figure 3**. Typical SNP images of subjects from the control and study group, as well as a schematic representation of detected nerve fibers used for characterization of the SNP at baseline and after 8 weeks of treatment.
**Figure 4**. Corneal nerve fiber length of patients in the group treated with high molecular weight hyaluronic acid (COMFORT SHIELD^{®} preservative-free sodium hyaluronate eye drops) and the control group at baseline and eight weeks. Figure 4 corresponds to Figure 1 with the inclusion of individual patient data points.

### DETAILED DESCRIPTION OF THE INVENTION

The present invention relates to a fluid comprising high molecular weight hyaluronic acid (HMWHA) for use in treating or preventing corneal nerve damage or loss in a human or non-human animal subject, wherein the fluid is for topical administration to the ocular surface of the subject, wherein the hyaluronic acid has an intrinsic viscosity of at least 2.5 m3/kg, wherein the hyaluronic acid has a molecular weight in the range of 3 million to 4 million Daltons, and wherein the subject has diabetes and diabetic peripheral neuropathy, diabetes but does not yet have diabetic peripheral neuropathy, diabetic corneal neuropathy, stroke, neurotrophic keratopathy (NK), or corneal nerve damage or loss caused by trauma or a medical procedure.

In one embodiment, the nerve damage or loss includes diminished nerve fiber length and/or diminished nerve fiber tortuosity.

In one embodiment, the corneal nerve damage or loss in the subject was identified by in vivo confocal microscopy (IVCM) on the eye of the subject.

In one embodiment, the subject has a sign or symptom of corneal nerve damage or loss. In one embodiment, the sign of corneal nerve damage or loss is one or more of the following: a decrease of corneal innervation or sensation, a reduction in the number of nerve fibers or bundles innervating the cornea, death of neurons innervating the cornea, a decrease or loss of neurotransmitter release, a decrease or loss of nerve growth factor release, abnormal tearing reflexes, abnormal blink reflexes, abnormal nerve morphology, appearance of abnormal nerve sprouts, abnormal tortuosity, increased bead-like nerve formations, thinning of nerve fiber bundles, thickening of nerve fiber bundles, diminished length of the inferior corneal nerve whorl, diminished density of corneal nerve, diminished length of corneal nerve, diminished branching of corneal nerve, recurrent corneal erosion, delayed epithelial wound healing of the cornea, or decreased tearing rate; or
wherein the symptom of corneal nerve damage or loss is one or more of the following: abnormal tear production or dryness, abnormal blinking, and difficulty or loss of ability to focus, decreased or lost visual acuity, or decreased or lost corneal sensitivity.

In one embodiment, the subject has mild, moderate, or severe neurotrophic keratopathy (NK); or wherein the NK is mild (stage 1) NK or moderate (stage 2) NK at the time of administration, and the HMWHA fluid prevent or delays the progression of the NK to a state of severe (stage 3) NK.

In one embodiment, the subject has type 1, type 2, or gestational diabetes and diabetic peripheral neuropathy; type 1, type 2, or gestational diabetes, but does not yet have diabetic peripheral neuropathy, or diabetic corneal neuropathy.

In one embodiment, the trauma is chemical or mechanical trauma; or corneal nerve damage or loss is caused by a medical procedure.

In one embodiment, the fluid is for administration in combination with one or more additional treatments before, during, or after topical administration of the HMWHA fluid, wherein the one or more additional treatments are selected from the group comprising: recombinant human nerve growth factor (cenegermin), matrix metalloproteinase inhibitor, plasma rich in growth factors (PRGF), therapeutic contact lens, temporary tarsorrhaphy (partial, or complete after HMWHA fluid is administered), amniotic membrane transplantation, penetrating keratoplasty, corneal transplantation, combination of cenegermin and corneal transplantation, or director or indirect corneal neurotization.

In one embodiment, the HMWHA fluid is a drop or a wash, such as a lavage; or
wherein the HMWHA fluid is administered to the ocular surface by a fluid delivery agent that is for topical administration to the ocular surface, preferably wherein the delivery agent is a particle that is coated with and/or secretes the fluid on to the ocular surface.

In one embodiment, the hyaluronic acid has an intrinsic viscosity in the range of 2.6 m3/kg to 2.9 m3/kg, or greater.

In one embodiment, the HMWHA fluid is preservative-free.

In one embodiment, the HMWHA fluid further comprises an additional glycosaminoglycan (GAG), an electrolyte such as sodium chloride, a buffer such as phosphate buffer, or a combination of two or more of the foregoing.

In one embodiment, the HMWHA fluid comprises HMWHA having a concentration of < 0.2 % w/v, preferably, wherein the HMWHA fluid comprises HMWHA having a concentration of 0.1 to 0.19 % w/v, and more preferably, wherein the HMWHA fluid comprises HMWHA having a concentration of about 0.15 % w/v.

In one embodiment, the HMWHA fluid has: a) a pH of 6.8 - 7.6; b) an osmolarity of 240 - 330 mOsmol/kg; c) a NaCl concentration of 7.6 - 10.5 g/l; and/or d) a phosphate concentration of 1.0 - 1.4 mmol/l.

Within the HYLAN M Study, a performance study of high molecular weight hyaluronic acid eye drops (COMFORT SHIELD^{®} preservative-free sodium hyaluronate eye drops, i.com medical GmbH, Munich Germany) in severe dry eyes, confocal microscopy is an optional diagnostic method to be performed by those investigators having the required instrumentation and experimentation at the baseline visit and the 8 weeks visit to analyze the sub-basal epithelial nerve plexus. Images from 16 patients were collected at baseline and after 8 weeks treatment. The images were from 8 patients of the control group who continued to use the control lubricant eye drops which they had been using by the time of inclusion in the HYLAN M study, and 8 patients for which the ocular lubricant eye drops had been substituted by COMFORT SHIELD^{®} eye drops.

To the inventors' surprise, a statistically significant (p = 0.031) growth of the total nerve fiber length in the patients treated with Comfort Shield eye drops was found (see Example 1 and Figure 1). This neurotrophic activity is an unexpected effect of high molecular weight hyaluronic acid (HMWHA), and is of great relevance for a number of ocular situations independent of dry eye disease. Further details concerning the HYLAN M study and the effects of HMWHA fluid, such as COMFORT SHIELD^{®} eye drops, on total nerve fiber length and the trophic effect at the subbasal nerve plexus are provided in van Setten et al. "The HYLAN M Study: Efficacy of 0.15% High Molecular Weight Hyaluronan Fluid in the Treatment of Severe Dry Eye Disease in a Multicenter Randomized Trial," J Clin Med., 2020 Nov 2; 9(11):3536; and van Setten et al., "High Molecular Weight Hyaluronan Promotes Corneal Nerve Growth in Severe Dry Eyes," J Clin Med., 2020 Nov 24; 9(12):3799.
The present invention concerns a fluid comprising high molecular weight hyaluronic acid (HMWHA) for use in treating or preventing corneal nerve damage or loss in a human or non-human animal subject, wherein the fluid is for topical administration to the ocular surface of the subject, wherein the hyaluronic acid has an intrinsic viscosity of at least 2.5 m³/kg, wherein the hyaluronic acid has a molecular weight in the range of 3 million to 4 million Daltons, and wherein the subject has diabetes and diabetic peripheral neuropathy, diabetes but does not yet have diabetic peripheral neuropathy, diabetic corneal neuropathy, stroke, neurotrophic keratopathy (NK), or corneal nerve damage or loss caused by trauma or a medical procedure. Without being limited by theory of mechanism of action, it is proposed that providing HMWHA to the ocular surface supports and promotes the restoration of the functional epithelial and stromal microenvironment that is conducive to axon outgrowth and nerve regeneration following a corneal nerve injury.

Hyaluronic acid (HA) is a carbohydrate - a glycosaminoglycan, specifically, which can be found in living organisms. The biological functions of endogenous HA include maintenance of the elastoviscosity of liquid connective tissues such as joint synovial fluid and eye vitreous fluid (Necas J et al., "Hyaluronic acid (hyaluronan): a review", Veterinarni Medicina, 2008, 53(8):397-411; Stern R et al., "Hyaluronan fragments: An information-rich system", European Journal of Cell Biology, 2006, 85:699-715). Although the specific mechanisms involved in the diverse signaling of HA are still poorly understood, it is known that HA can modulate multi-faceted biological effects that can vary depending on HA size (Cyphert JM et al., "Size Matters: Molecular Weight Specificity of Hyaluronan Effects in Cell Biology," International Journal of Cell Biology, 2015, Epub 2015 Sep 10, 563818).

Sodium hyaluronate and other viscoelastic agents have been used in intraocular surgery since the 1970s and for treatment of dry eyes since the 1980s (Higashide T and K Sugiyama, "Use of viscoelastic substance in ophthalmic surgery- focus on sodium hyaluronate," Clinical Ophthalmology, 2008, 2(1):21-30; Polack FM and MT McNiece, "The treatment of dry eyes with Na hyaluronate (Healon) - preliminary report, 1982, 1(2):133-136); however, little attention has been paid thus far to the biological function of hyaluronic acid in epithelia (Miller-Lierheim WGK, "Tränenersatzlösungen, Neues über Hyaluronsäure," Aktuelle Kontaktologie, April 2015, 17-19).

The high molecular weight hyaluronic acid or "HMWHA" used in the invention refers to hyaluronic acid having an intrinsic viscosity of at least 2.5 m³/kg (2.5 m³/kg or greater) as determined by the method of the European Pharmacopoeia 9.0, "Sodium Hyaluronate", page 3584. Briefly, the intrinsic viscosity [η] is calculated by linear least-squares regression analysis using the Martin equation: Log₁₀ (nᵣ-1/c) = log₁₀ [η] + κ[η]*c*. In some embodiments, the high molecular weight hyaluronic acid has an intrinsic viscosity of at least 2.9 m³/kg (2.9 m³/kg or greater).

In some embodiments, the hyaluronic acid has a concentration of < 0.2 % w/v. In some embodiments, the hyaluronic acid has a concentration of 0.1 to 0.19 % w/v. In some embodiments, the hyaluronic acid has a concentration of 0.15 % w/v.

In some embodiments, the fluid has: a) a pH of 6.8 - 7.6; b) an osmolarity of 240 - 330 mOsmol/kg; c) a NaCl concentration of 7.6 - 10.5 g/l; and/or d) a phosphate concentration of 1.0 - 1.4 mmol/l.

In some embodiments, the fluid is a clear and colorless solution, free from visible impurities. It is envisaged that the fluid is sterile.

In some embodiments, the fluid according to the invention is COMFORT SHIELD^{®} preservative-free sodium hyaluronate eye drops.

According to the invention, the HA has a molecular weight in the range of 3 million to 4 million Daltons. The HA may have a molecular weight of at least 3 million Daltons as calculated by the Mark-Houwink equation. In some embodiments, the HA has a molecular weight in the range of 3 million to 4 million Daltons as calculated by the Mark-Houwink equation.

In some embodiments, the high molecular weight HA is hyaluronan. In some embodiments, the high molecular weight HA is cross-linked. In some embodiments, the high molecular weight HA is non-cross-linked. In some embodiments, the high molecular weight HA is linear. In some embodiments, the high molecular weight HA is non-linear (e.g., branched). In some embodiments, the high molecular weight HA is a derivative of hyaluronan, such as an ester derivative, amide derivative, or sulfated derivative, or a combination of two or more of the foregoing.

The fluid may be administered to the ocular surface of one or both eyes of the subject by any topical administration method. For example, the fluid may be administered as one or more drops from a device for dispensing eye drops, such as an eye dropper. The fluid may be self-administered or administered by a third party. The dosage administered, as single or multiple doses, to an ocular surface will vary depending upon a variety of factors, including patient conditions and characteristics, extent of symptoms, concurrent treatments, frequency of treatment and the effect desired. For example, one or more drops (of, for example, about 30 microliters each) may be administered.

While administration of 1-3 drops, one to three times per day, may be sufficient under some circumstances, more frequent topical administration is likely to be needed for most cases of corneal nerve damage or loss *e.g.,* 1-3 drops for four, five, six, seven, eight, nine, ten, or more times per day. In some embodiments, 3 or more drops are administered one or more times per day.

In some embodiments, the frequency of administration and/or the amounts per dose can be decreased with time, as the corneal nerve regenerates. For example, in some cases, after four weeks, the amount administered may be reduced and/or the frequency of administrations each day may be reduced or the frequency of administrations may be reduced to semi-daily. However, while eye drops are typically taken by a subject for a frequency and duration "as needed", loss of sensation may be result from corneal nerve damage or loss, making the subject's self-assessment of their needs as to frequency and duration unreliable. Therefore, administration of the fluid should not be discontinued merely based on absence of discomfort, to avoid stopping treatment prematurely.

The corneal nerve damage or loss to be treated using the HMWHA fluid and method of the invention can be any type of nerve injury that inhibits or impairs normal corneal nerve turnover, growth, function, or any combination of two or more of the foregoing, and may involve any extent of damage to the nerve structure (architecture and/or continuity) and, in some cases, surrounding tissue. The nerve injury may be any type or stage of injury, such as neurapraxia, axonotmesis, or neurotmesis (Seddon HJ, "A Classification of Nerve Injuries", British Medical Journal, 1942, 2(4260): 237-9). For example, the nerve damage or loss may include one or more of the following characteristics: diminished nerve fiber length, diminished nerve fiber tortuosity, diminished nerve fiber density, and complete or partial nerve fiber severance.

The HMWHA fluid is topically administered to the ocular surface of the eye in an amount, and for a duration of time, sufficient to diminish the net loss of corneal nerve from the corneal nerve damage or loss relative to the net loss of corneal nerve that would occur in the absence of the HMWHA fluid. Thus, sufficient HMWHA fluid is topically administered to lessen the net loss of corneal nerve that would otherwise occur without treatment with the HMWHA fluid.

The treatment method may include a step of identifying the subject as one having corneal nerve damage or loss prior to topical administration of the HMWHA fluid. Preferably, the subject is identified using *in vivo* confocal microscopy (IVCM) on the eye or eyes having the nerve damage or loss (Alhatem A et al., "In vivo confocal microscopy in dry eye disease and related conditions," Seminars in Ophthalmology, 2012, vol. 27, pp. 138-148; Patel DV and C. N. McGhee, "Quantitative analysis of in vivo confocal microscopy images: a review," Survey of Ophthalmology, 2013, vol. 58, pp. 466-475; Cruzat A. et al., "In vivo confocal microscopy of corneal nerves in health and disease," The Ocular Surface, vol. 15, pp. 15-47, 2017).

Optionally, the subject is monitored one or more times using IVCM during and/or after treatment, and confocal microscopic images can be compared to previous images to assess status and progress of corneal nerve healing and growth.

Optionally, the method includes a step of, prior to administration of the HMWHA fluid, identifying the subject as one having one or more signs or symptoms of corneal nerve damage or loss. Examples of signs of corneal nerve damage or loss include, but are not limited to: a decrease of corneal innervation or sensation, a reduction in the number of nerve fibers or bundles innervating the cornea, death of neurons innervating the cornea, a decrease or loss of neurotransmitter release, a decrease or loss of nerve growth factor release, abnormal tearing reflexes, abnormal blink reflexes, abnormal nerve morphology, appearance of abnormal nerve sprouts, abnormal tortuosity, increased bead-like nerve formations, thinning of nerve fiber bundles, thickening of nerve fiber bundles, diminished length of the inferior corneal nerve whorl, diminished density of corneal nerve, diminished length of corneal nerve, diminished branching of corneal nerve, recurrent corneal erosion, delayed epithelial wound healing of the cornea, and decreased tearing rate. Examples of symptoms of corneal nerve damage or loss include, but are not limited to: abnormal tear production or dryness, abnormal blinking, and difficulty or loss of ability to focus, decreased or lost visual acuity, and decreased or lost corneal sensitivity.
According to the invention, the subject to be administered the fluid for use of the invention has diabetes and diabetic peripheral neuropathy, diabetes but does not yet have diabetic peripheral neuropathy, diabetic corneal neuropathy, stroke, neurotrophic keratopathy (NK), or corneal nerve damage or loss caused by trauma or a medical procedure.

In some embodiments, the corneal nerve damage or loss is caused by disease, trauma (chemical, mechanical, etc.), congenital defect, or medical procedure.

In some embodiments, the corneal nerve damage or loss comprises an impairment of corneal innervation caused by a viral infection, medicamentosa, chronic contact lens use, surgery, diabetes (type 1, type 2, or gestational), or multiple sclerosis.

According to the invention, the subject may have neurotrophic keratopathy (mild, moderate, or severe NK) in the eye at the time of administration, and the HMWHA fluid alleviates one or more signs or symptoms of the NK. In some embodiments, the NK is mild NK (also known as stage 1) or moderate NK (also known as stage 2) at the time of administration, and the HMWHA fluid prevent or delays the progression of the NK to a state of severe NK (also known as stage 3). In some embodiments, the NK is severe NK.

In some embodiments, the subject does not have NK at the time of administration, and the HMWHA fluid prevents or delays the onset of NK.

In some embodiments, the subject has an ocular surface disease or disorder (mild, moderate, or severe). In other embodiments, the subject does not have an ocular surface disease or disorder. Examples of ocular surfaces diseases or disorders, and their pathogenesis, can be found in Belmonte C, "Pain, Dryness, and Itch Sensations in Eye Surface Disorders are Defined By A Balance Between Inflammation and Sensor Nerve Injury," Cornea, 2019, 38 Suppl 1:S11-S24. In some embodiments, the subject has dry eye disease (mild, moderate, or severe). In other embodiments, the subject does not have dry eye disease. In some embodiments, the subject has a tear film deficiency. In other embodiments, the subject does not have a tear film deficiency.

In some embodiments, the subject has a tear volume deficiency. In some embodiments, the subject does not have a tear volume deficiency; however, the subject has an ocular surface abnormality (a topographic anomaly) comprising elevations on the cornea or elsewhere on the eye surface for which the normal tear film (tear film of normal surface tension and viscosity) does not cover, resulting in areas of friction at the ocular surface (van Setten, Epitheliopathy of the bleb (EoB)-identifying attrition: A new model for failure of glaucoma surgery, New Frontiers in Ophthalmology, 2018: 4(3): 1-4).

Diabetes mellitus is a common metabolic disease known to cause structural and functional changes in the human cornea, with ocular complications from diabetes mellitus, including corneal nerve and loss, frequently occurring. According to the invention, the subject treated with the method of the invention may have diabetes mellitus (type 1, type 2, or gestational).

In some embodiments, the subject has diabetes (type 1, type 2, or gestational) and has diabetic peripheral neuropathy.

In some embodiments, the subject has diabetic corneal neuropathy, and the HMWHA fluid reverses the diabetic corneal neuropathy.

In some embodiments, the subject has diabetes (type 1, type 2, or gestational) but does not yet have diabetic peripheral neuropathy.

In some embodiments, the subject has a genetic condition known to be comorbid with ocular surface damage that may lead to NK (such as Riley-Day syndrome (familial dysautonomia), Goldenhar-Gorlin syndrome, Mobius syndrome, and familial corneal hypoaestesia).

In some embodiments, the subject has a systemic condition known to be comorbid with ocular surface damage that may lead to NK (such as diabetes mellitus (type 1, type 2, or gestational), leprosy, vitamin A deficiency, amyloidosis, and multiple sclerosis).

In some embodiments, subject has a condition of the central nervous system (CNS) known to cause ocular surface damage that may lead to NK (such as neoplasm, aneurysm, stroke, degenerative disorder of the CNS (such as Alzheimer's disease and Parkinson's disease), and post-neurosurgical procedure (such as for acoustic neuroma, trigeminal neuralgia, or other surgical injury to the trigeminal nerve).

In some embodiments, the subject has an ocular condition known to cause ocular surface damage that may lead to NK (such as post-herpes infection (herpes simplex and herpes zoster), other infection with nerve damage related to keratoneuritis, chemical or physical burn, abuse of topical anesthetic, drug toxicity (such as trimolol, betaxolol, diclofenac sodium, sulphacetamide 30%), chronic ocular surface injury or inflammation, ocular surgery (such as cataract surgery, glaucoma surgery, laser in situ keratomileusis (LASIK), and photoreactive keratectomy (PRK), penetrating keratoplasty (PK), deep anterior lamellar keratoplasty (DALK), collagen crosslinking for keratoconus, vitrectomy for retinal detachment, photocoagulation to treat diabetic retinopathy, postsurgical or laser treatment), contact lens wearing, orbital neoplasia, or corneal dystrophy (lattice or granular).

In some embodiments, the method further comprises administering one or more additional treatments before, during, or after topically administering the HMWHA fluid, selected from among: recombinant human nerve growth factor (cenegermin), matrix metalloproteinase inhibitor, plasma rich in growth factors (PRGF), therapeutic contact lens, temporary tarsorrhaphy (partial, or complete after HMWHA fluid is administered), amniotic membrane transplantation, penetrating keratoplasty, corneal transplantation, combination of cenegermin and corneal transplantation, or director or indirect corneal neurotization. Optionally, if the additional treatment involves administration of an active agent, it may be administered in the HMWHA fluid or in a separately administered formulation.

In some embodiments, the HMWHA fluid is administered directly to the ocular surface as drops or as a wash (*e.g*., lavage).

In some embodiments, 1 to 3 drops are administered, 1 to 3 times per day.

In some embodiments, 1 to 3 drops are administered, 4, 5, 6, 7, 8, 9, or 10 or more times per day.

In some embodiments, 3 or more drops are administered one or more times per day.

In some embodiments, HMWHA fluid is administered indirectly to the ocular surface by a delivery agent (a fluid delivery agent) that is topically administered to the ocular surface or other part of the eye (*e.g.,* a particle that is coated with and/or secretes the fluid on to the ocular surface).

In some embodiments, the hyaluronic acid has an intrinsic viscosity in the range of 2.6 m³/kg to 2.9 m³/kg, or greater.

In some embodiments, the hyaluronic acid has a molecular weight of at least 3 million Daltons. In some embodiments, the hyaluronic acid has a molecular weight in the range of 3 million to 4 million Daltons.

In some embodiments, the HMWHA fluid comprises HMWHA having a concentration of < 0.2 % w/v. In some embodiments, the HMWHA fluid comprises HMWHA having a concentration of 0.1 to 0.19 % w/v. In some embodiments, the HMWHA fluid comprises HMWHA having a concentration of about 0.15 % w/v.

In some embodiments, the HMWHA fluid has the following composition/characteristics, which correspond to those of COMFORT SHIELD^{®} preservative-free sodium hyaluronate eye drops:
a) a pH of 6.8 - 7.6;
b) an osmolarity of 240 - 330 mOsmol/kg;
c) a NaCl concentration of 7.6 - 10.5 g/l; and/or
d) a phosphate concentration of 1.0 - 1.4 mmol/l.

In some embodiments, the HMWHA fluid is a clear and colorless solution, free from visible impurities.

In some embodiments, the HMWHA fluid is sterile.

In some embodiments, the HMWHA fluid is COMFORT SHIELD^{®} preservative-free sodium hyaluronate eye drops.

In some embodiments, the HMWHA fluid contains no other bioactive agent *(e.g.,* no hydrophobic active ingredient). In other embodiments the HMWHA fluid further comprises a bioactive agent (*e.g.,* a hydrophobic active ingredient). As used herein, the term "bioactive agent" refers to any substance that has an effect on the human or non-human animal subject when administered in an effective amount to affect the tissue. The bioactive agent may be any class of substance such as a drug molecule or biologic (*e.g*., polypeptide, carbohydrate, glycoprotein, immunoglobulin, nucleic acid), may be natural products or artificially produced, and may act by any mechanism such as pharmacological, immunological, or metabolic. Examples of classes of bioactive agents include substances that modify the pressure of the eye (*e.g.,* enzyme inhibitors) and anti-angiogenic agents. Some specific examples of bioactive agents include steroids (*e.g*., corticosteroids), antibiotics, immunosuppressants, immunomodulatory agents, tacrolimus, plasmin activator, anti-plasmin, and cyclosporin A. In some embodiments, the bioactive agent is a steroid or antibiotic to treat or prevent eye infection; glaucoma drug such as prostaglandin analog, beta blocker, alpha agonist, or carbonic anhydrase inhibitor; agent for allergy eye relief such as histamine antagonist or non-steroidal anti-inflammatory drug; or mydriatic agent. Unfortunately, in some cases, the bioactive agent or agents included in the fluid may be irritative or damaging to the eye (*e.g.*, cyclosporin A). Advantageously, through its rheological property and other properties, the high molecular weight HA in the fluid can alleviate and/or protect the eye from the irritative and/or damaging effects of the biologically active agent or agents within the fluid (*i.e.,* the bioactive agent would be more irritative or more damaging to the eye if administered without the high molecular weight HA).

In some embodiments, the HMWHA fluid contains no steroid, antibiotic or immunomodulator. In some embodiments, the fluid contains no other bioactive agent (*e.g.,* no hydrophobic active ingredient).

In some embodiments, the HMWHA fluid includes a preservative and/or detergent, preferably one that does not cause damage or irritation to the eye. In other embodiments, the HMWHA fluid does not include a preservative or detergent (*i.e.,* the fluid is preservative-free and detergent-free). In some circumstances, it may be desirable to include one or more preservatives or detergents within the fluid. Often, such preservatives and detergents are irritative or damaging to the eye. Advantageously, through its rheological property and other properties, the fluid can alleviate and/or protect the eye from the irritative and/or damaging effects of the preservative or detergent within the fluid. Thus, in some embodiments, the fluid further comprises a preservative or detergent that is irritative or damaging to the eye (*i.e.,* a preservative or detergent that would be more irritative or more damaging to the eye if administered without the high molecular weight HA).

In some embodiments, the HMWHA fluid includes cyclosporin A, cetalkoniumchloride, tyloxapol, or a combination of two or more of the foregoing.

In some embodiments, the HMWHA fluid is administered to the subject before, during, and/or after administration of another composition comprising a bioactive agent to the subject. In some circumstances, it may be desirable to include one or more preservatives or detergents within the other composition. As indicated above, often, such preservatives and detergents are irritative or damaging to the eye, and some bioactive agents themselves may be irritative or damaging to the eye. Advantageously, through its rheological property and other properties, the fluid can alleviate and/or protect the eye from the irritative and/or damaging effects of the bioactive agent, preservative, and/or detergent within the other composition. Thus, the bioactive agent, preservative, and/or detergent within the other composition would be more irritative or more damaging to the eye if administered without the fluid.

In some embodiments, the other composition includes one or more of an antibiotic, immunosuppressant, or immunomodulatory agent.

In some embodiments, the other composition includes cyclosporin A, cetalkoniumchloride, tyloxapol, or a combination of two or more of the foregoing.

The other composition administered to the subject may be in any form and administered by any route (*e.g.,* local or systemic). In some embodiments, the other composition is administered to the eye, *e.g.,* topically or by injection. In some embodiments, the other composition is topically administered to the ocular surface.

In some embodiments, the preservative or detergent included in the HMWHA fluid or other composition is a chemical preservative or oxidative preservative.

In some embodiments, the preservative or detergent included in the HMWHA fluid or other composition is one that kills susceptible microbial cells by disrupting the lipid structure of the microbial cell membrane, thereby increasing microbial cell membrane permeability.

In some embodiments, the preservative or detergent included in the HMWHA fluid or other composition is one that normal causes damage to the corneal tissues, such as corneal epithelium, endothelium, stroma, and interfaces such as membranes, but the HMWHA fluid is ameliorates or is protective against the damage..

In some embodiments, the preservative or detergent included in the HMWHA fluid or other composition is selected from the group consisting of quaternary ammonium preservative (*e.g.*, benzalkonium chloride (BAK) or cetalkoniumchloride), chlorobutanol, edetate disodium (EDTA), polyquaternarium-1 (*e.g.,* Polyquad^{™} preservative), stabilized oxidizing agent (*e.g.,* stabilized oxychloro complex (*e.g.,* Purite^{™} preservative)), ionic-buffered preservative (*e.g*., sofZia^{™} preservative), polyhexamethylene biguanide (PHMB), sodium perborate (*e.g*., GenAqua^{™} preservative), tylopaxol, and sorbate.

In some embodiments, the HMWHA fluid is at least essentially mucin-free; or in other words having a mucin concentration of < 0.3 % w/v.

In some embodiments, the HMWHA fluid further includes a glycosaminoglycan (GAG), *i.e.,* includes one or more GAGs in addition to the high molecular weight HA; electrolyte (*e.g.,* sodium chloride); buffer (*e.g.,* phosphate buffer); or a combination of two or more of the foregoing.

The HMWHA fluid may be used in conjunction with a bandage contact lens. Thus, the method may further include applying a bandage contact lens to the eye before, during, and/or after administering the fluid. For example, the fluid may be administered before applying the bandage contact lens, after the contact lens, and/or placing fluid on the bandage contact lens before applying the bandage contact lens to the eye. Use of the fluid allows the bandage contact lens to exert pressure on the ocular surface while simultaneously minimizing friction at the ocular surface. Advantageously, the fluid and bandage contact lens can safely be used shortly after ocular surgery, *e.g*., glaucoma surgery.

Also described herein is a kit that may be used for carrying out the method of the invention described herein, i.e., treating corneal nerve damage or loss. The kit comprises the HMWHA fluid described herein, and one or more bandage contact lenses. Bandage contact lenses may be packaged together with the fluid within the same container (with the bandage contact lenses in contact with the fluid), or the bandage contact lenses may be separate from the fluid, packaged in separate containers. Suitable containers include, for example, bottles, vials, syringes, blister pack, *etc.* The containers may be formed from a variety of materials such as glass or plastic.

The kit may include a delivery agent (separately or in association with the fluid) that is to be brought into contact with the ocular surface or other part of the eye. For example, the kit may include particles (*e.g*., microparticles or nanoparticles) that are coated with the fluid and/or release the fluid onto the ocular surface.

Optionally, the kit may include a device for dispensing eye drops (*e.g.,* an eye dropper), which may or may not serve as a container for the HMWHA fluid in the kit before the kit's outer packaging is accessed (*e.g.,* opened), *i.e.,* the eye drop dispensing device may function to contain the fluid provided in the unaccessed (unopened) kit, or may be empty and receive the fluid after the kit is accessed. Optionally, the kit may include a label or packaging insert with printed or digital instructions for use of the kit, *e.g.,* for carrying out the method of the invention.

Kits can include packaging material that is compartmentalized to receive one or more containers such as vials, tubes, and the like, each of the container(s) including one of the separate elements to be used in a method described herein. Packaging materials for use in packaging pharmaceutical products include, by way of example only U.S. Pat. Nos. 5,323,907, 5,052,558 and 5,033,252. Examples of pharmaceutical packaging materials include, but are not limited to, blister packs, bottles, tubes, pumps, bags, vials, light-tight sealed containers, syringes, bottles, and any packaging material suitable for a selected formulation and intended mode of administration and treatment.

A kit may include one or more additional containers, each with one or more of various materials desirable from a commercial and user standpoint for use of the compositions described herein. Non-limiting examples of such materials include, but not limited to, buffers, diluents, carrier, package, container, vial and/or tube labels listing contents and/or instructions for use, and package inserts with instructions for use.

A label can be on or associated with the container. A label can be on a container when letters, numbers or other characters forming the label are attached, molded or etched into the container itself; a label can be associated with a container when it is present within a receptacle or carrier that also holds the container, *e.g*., as a package insert. A label can be used to indicate that the contents are to be used for a specific therapeutic application. The label can also indicate directions for use of the contents, such as in the methods described herein.

For example, the HMWHA fluid can be presented in a pack or dispenser device which can contain one or more unit dosage forms containing a composition disclosed herein. The pack can for example contain metal or plastic foil, such as a blister pack. The pack or dispenser device can be accompanied by instructions for administration.

### HMWHA Fluid Preparation

As indicated above, the hyaluronic acid of the fluid has an intrinsic viscosity of at least 2.5 m³/kg, and preferably a concentration of < 0.2 % w/v. In some embodiments, the hyaluronic acid has an intrinsic viscosity of at least 2.9 m³/kg.

Viscoelasticity is defined as characteristics of a fluid having both viscous and elastic properties. The zero shear viscosity is determined as the steady shear plateau viscosity at vanishing shear rate. For highly viscous formulations, measurement with a controlled stress rheometer is preferred.

The relation between molecular weight and intrinsic viscosity [η] in m³/kg is given through the Mark-Houwink equation: $\left[η\right] = k ⋅ {\left({M}_{rm}\right)}^{a}$
with Mᵣₘ being the molecular mass in MDa
and the coefficients $k = 1.3327 ⋅ {10}^{- 4}$ and $a = 0.6691$
which values for k and a having been found as most predictive.

The HMWHA fluid may be produced by: sterilizing the filling line; adding purified water or water for injection (WFI) to a stainless steel mixing tank; adding salts while mixing; slowly adding HA and mixing until a homogeneous solution/fluid is achieved; adjusting pH value by adding NaOH or HCl, if required, while continuing the mixing process; transferring the solution over a 1 µm pore size filter cartridge to a sterile holding tank; and aseptically filling the solution via sterile filtration into the sterile primary package (monodose or vial). In the case of monodoses, this may be done by a blow-fill-seal (BFS) process.

Preferably, the HMWHA fluid has at least essentially mucin-free or in other words having a mucin concentration of < 0.3 % w/v. This means that the flow behavior or properties essentially is reached or adjusted by hyaluronan and not by mucin naturally present in the subject's tear fluid and mainly responsible for the flow behavior thereof.

It is preferred that if substances are added that increase the viscosity, they are added towards, or during, or as a final step. The mixing is carried out so as to reach a homogeneous mixture. As an alternative or in addition, it is preferred to initially provide purified water or water for injection as a basis, and then, optionally, electrolytes, buffers and substances which do not increase the viscosity are added at first to the purified water or water for injection.

HA is further described in the monograph of the European Pharmacopoeia 9.0, page 3583 (Sodium Hyaluronate).

In one embodiment, the fluid used in the method and kit of the invention has the characteristics listed in Table 1:

**Table 1.**

| **Characteristic** | **Specification** | **Test Method** |
|---|---|---|
| Appearance | clear and colorless solution, free from visible impurities | Ph.Eur. |
| pH value | 6.8 - 7.6 | Ph.Eur. |
| Osmolality | 240 - 330 mosmol/kg | Ph.Eur. |
| HA concentration | 0.10 - 0.19 % w/v | Ph.Eur. |
| NaCl concentration | 7.6 - 10.5 g/l | Ph.Eur. |
| Sterility | Sterile | Ph.Eur. |
| Phosphate concentration | 1.0 - 1.4 mmol/l | Ph.Eur. |

### Definitions

The term "a," "an," "the" and similar terms used in the context of the present invention (especially in the context of the claims) are to be construed to cover both the singular and plural unless otherwise indicated herein or clearly contradicted by the context. Thus, for example, reference "a cell" or "a compound" should be construed to cover both a singular cell or singular compound and a plurality of cells and a plurality of compounds unless indicated otherwise or clearly contradicted by the context. Similarly, the word "or" is intended to include "and" unless the context clearly indicates otherwise. The abbreviation, *"e.g."* is derived from the Latin *exempli gratia,* and is used herein to indicate a non-limiting example. Thus, the abbreviation "e.g." is synonymous with the term "for example." Furthermore, to the extent that the terms "including", "includes", "having", "has", "with", or variants thereof are used in either the detailed description and/or the claims, such terms are intended to be inclusive in a manner similar to the term "comprising". The transitional terms/phrases (and any grammatical variations thereof) "comprising", "comprises", "comprise", "consisting essentially of", "consists essentially of", "consisting of" and "consists of" can be used interchangeably.

The term "effective amount" in the context of the administered fluid of the invention means the amount of fluid necessary to obtain a desired result, such as the amount necessary to diminish the net loss of corneal nerve from the corneal nerve damage or loss relative to the net loss of corneal nerve that would occur in the absence of the HMWHA fluid.

The term "isolated," when used as a modifier of a composition, means that the compositions are made by human intervention or are separated from their naturally occurring *in vivo* environment. Generally, compositions so separated are substantially free of one or more materials with which they normally associate with in nature, for example, one or more protein, nucleic acid, lipid, carbohydrate, cell membrane. A "substantially pure" molecule can be combined with one or more other molecules. Thus, the term "substantially pure" does not exclude combinations of compositions. Substantial purity can be at least about 60% or more of the molecule by mass. Purity can also be about 70% or 80% or more, and can be greater, for example, 90% or more. Purity can be determined by any appropriate method, including, for example, UV spectroscopy, chromatography (e.g., HPLC, gas phase), gel electrophoresis (*e.g.*, silver or coomassie staining) and sequence analysis (for nucleic acid and peptide).

As used herein, the term "hyaluronic acid" (HA) refers to the glycosaminoglycan composed of disaccharide repeats of N-acetylglucosamine and glucuronic acid found in nature, also known as hyaluronan (*e.g*., the straight chain, glycosaminoglycan polymer composed of repeating units of the disaccharide [-D-glucuronic acid-b1,3-N-acetyl-D-glucosamine-b1,4-]*n*), as well as derivatives of hyaluronan having chemical modifications such as esters of hyaluronan, amide derivatives, alkyl-amine derivatives, low molecular weight and high molecular weight forms of hyaluronans, and cross-linked forms such as hylans. Thus, the disaccharide chain may be linear or non-linear. Hyaluronan can be cross-linked by attaching cross-linkers such as thiols, methacrylates, hexadecylamides, and tyramines. Hyaluronan can also be cross-linked directly with formaldehyde and divinylsulfone. Examples of hylans include, but are not limited to, hylan A, hylan A, hylan B, and hylan G-F 20 (Hargittai M and I Hargittai, "More Conversations with Hyaluronan Scientists," from Hyaluronan - From Basic Science to Clinical Applications, Balazs EA, Ed., Vol. 3, 2011, PubMatrix, Edgewater, NJ; Cowman MK et al., Carbohydrate Polymers 2000, 41:229-235; Takigami S et al., Carbohydrate Polymers, 1993, 22:153-160; Balazs EA et al., "Hyaluronan, its cross-linked derivative-Hylan-and their medical applications", in Cellulosics Utilization: Research and Rewards in Cellulosics, Proceedings of Nisshinbo International Conference on Cellulosics Utilization in the Near Future (Eds Inagaki, H and Phillips GO), Elsevier Applied Science (1989), NY, pp.233-241; Koehler L et al., Scientific Reports, 2017, 7, article no. 1210; and Pavan M et al., Carbohydr Polym, 2013, 97(2): 321-326).

The term "hyaluronic acid" or HA includes HA itself and pharmaceutically acceptable salts thereof. The HA can be formulated into pharmaceutically-acceptable salt forms. Pharmaceutically-acceptable salts of HA can be prepared using conventional techniques.

The term "high molecular weight" or "HMW" in the context of hyaluronic acid of the invention refers to hyaluronic acid having an intrinsic viscosity of at least 2.5 m³/kg (2.5 m³/kg or greater) as determined by the method of the European Pharmacopoeia 9.0, "Sodium Hyaluronate", page 3584. Briefly, the intrinsic viscosity [η] is calculated by linear least-squares regression analysis using the Martin equation: Log₁₀ (nᵣ-1/c) = log₁₀ [η] + κ[η]*c*. In some embodiments, the high molecular weight hyaluronic acid has an intrinsic viscosity of at least 2.9 m³/kg.

The terms "nerve damage or loss" (in the cornea) and "corneal nerve damage or loss" are used interchangeably herein to refer to any type of nerve injury that inhibits or impairs normal corneal nerve turnover, orientation, growth, function, or any combination of two or more of the foregoing, and may involve any extent of damage to the nerve structure (architecture and/or continuity) and, in some cases, surrounding tissue. The nerve injury may be any type or stage of injury, such as neurapraxia, axonotmesis, or neurotmesis (Seddon HJ, "A Classification of Nerve Injuries", British Medical Journal, 1942, 2(4260): 237-9). For example, the nerve damage or loss may include one or more of the following characteristics: diminished nerve fiber length, diminished nerve fiber tortuosity, diminished nerve fiber density, and complete or partial nerve fiber severance. The nerve damage or loss may be the result of any type of influence that reduces neuronal function (a neurotrophic injury) and/or a type that causes a complete absence of neuronal function (a neuroparalytic injury), such as the case in nerve severance.

The term "neurotrophic keratopathy" or "NK" refers to a degenerative corneal disease caused by impaired corneal innervation, and characterized by a reduction or absence of corneal sensation, which may result in epithelial keratopathy, epithelial defect, stromal ulceration, and eventually corneal perforation. Ocular or systemic conditions that alter normal corneal innervation may result in NK. Potential etiological conditions include, for example, infection such as herpes keratitis (zoster and simplex); topical anesthetic abuse; chemical and physical burns; contact lens abuse (chronic contact lens wearing); topical drug toxicity; irradiation to eye or adnexa; chronic use of topical medications containing benzalkonium chloride (BAK); corneal procedures such as laser *in situ* keratomileusis (LASIK), photorefractive keratectomy (PRK), corneal transplantation surgery (particularly penetrating keratoplasty (PK) and deep anterior lamellar keratoplasty (DALK)), and collagen crosslinking of keratoconus eyes; non-corneal ocular procedures such as vitrectomy for retinal detachment and photocoagulation to treat diabetic retinopathy, and indirect laser for proliferative diabetic retinopathy; and non-ocular etiologies such as neurosurgical procedures or trauma damaging the fifth cranial nerve, stroke, aneurisms, multiple sclerosis, intracranial masses, diabetes, leprosy, vitamin A deficiency, drugs (narcoleptics and antipsychotics), and congenital hypoplasia of the trigeminal nerve.

As used herein, the term "ocular surface" refers to the cornea and conjunctiva, and portions thereof, including the conjunctiva covering the upper and lower lids. The HMWHA fluid may be topically administered to one or more parts of the ocular surface, including, for example, the entire ocular surface.

"Pharmaceutically acceptable salt" includes both acid and base addition salts. A pharmaceutically acceptable salt of HA or any one of the other compounds described herein is intended to encompass any and all pharmaceutically suitable salt forms. Preferred pharmaceutically acceptable salts described herein are pharmaceutically acceptable acid addition salts and pharmaceutically acceptable base addition salts.

"Pharmaceutically acceptable acid addition salt" refers to those salts which retain the biological effectiveness and properties of the free bases, which are not biologically or otherwise undesirable, and which are formed with inorganic acids such as hydrochloric acid, hydrobromic acid, sulfuric acid, nitric acid, phosphoric acid, hydroiodic acid, hydrofluoric acid, phosphorous acid, and the like. Also included are salts that are formed with organic acids such as aliphatic mono- and dicarboxylic acids, phenyl-substituted alkanoic acids, hydroxy alkanoic acids, alkanedioic acids, aromatic acids, aliphatic and. aromatic sulfonic acids, etc. and include, for example, acetic acid, trifluoroacetic acid, propionic acid, glycolic acid, pyruvic acid, oxalic acid, maleic acid, malonic acid, succinic acid, fumaric acid, tartaric acid, citric acid, benzoic acid, cinnamic acid, mandelic acid, methanesulfonic acid, ethanesulfonic acid, p-toluenesulfonic acid, salicylic acid, and the like. Exemplary salts thus include sulfates, pyrosulfates, bisulfates, sulfites, bisulfites, nitrates, phosphates, monohydrogenphosphates, dihydrogenphosphates, metaphosphates, pyrophosphates, chlorides, bromides, iodides, acetates, trifluoroacetates, propionates, caprylates, isobutyrates, oxalates, malonates, succinate suberates, sebacates, fumarates, maleates, mandelates, benzoates, chlorobenzoates, methylbenzoates, dinitrobenzoates, phthalates, benzenesulfonates, toluenesulfonates, phenylacetates, citrates, lactates, malates, tartrates, methanesulfonates, and the like. Also contemplated are salts of amino acids, such as arginates, gluconates, and galacturonates (see, for example, Berge S. M. et al., "Pharmaceutical Salts," Journal of Pharmaceutical Science, 66:1-19 (1997)). Acid addition salts of basic compounds may be prepared by contacting the free base forms with a sufficient amount of the desired acid to produce the salt according to methods and techniques with which a skilled artisan is familiar.

"Pharmaceutically acceptable base addition salt" refers to those salts that retain the biological effectiveness and properties of the free acids, which are not biologically or otherwise undesirable. These salts are prepared from addition of an inorganic base or an organic base to the free acid. Pharmaceutically acceptable base addition salts may be formed with metals or amines, such as alkali and alkaline earth metals or organic amines. Salts derived from inorganic bases include, but are not limited to, sodium, potassium, lithium, ammonium, calcium, magnesium, iron, zinc, copper, manganese, aluminum salts and the like. Salts derived from organic bases include, but are not limited to, salts of primary, secondary, and tertiary amines, substituted amines including naturally occurring substituted amines, cyclic amines and basic ion exchange resins, for example, isopropylamine, trimethylamine, diethylamine, triethylamine, tripropylamine, ethanolamine, diethanolamine, 2-dimethylaminoethanol, 2-diethylaminoethanol, dicyclohexylamine, lysine, arginine, histidine, caffeine, procaine, N,N-dibenzylethylenediamine, chloroprocaine, hydrabamine, choline, betaine, ethylenediamine, ethylenedianiline, N-methylglucamine, glucosamine, methylglucamine, theobromine, purines, piperazine, piperidine, N-ethylpiperidine, polyamine resins and the like. See Berge *et al*., *supra.* In some embodiments, the pharmaceutically acceptable salt is sodium salt (see "Sodium Hyaluronate" at page 3583 of European Pharmacopoeia 9.0).

As used herein, the terms "subject", "patient", and "individual" refer to a human or non-human animal. A subject also refers to, for example, primates (*e.g.*, humans), cows, sheep, goats, horses, dogs, cats, rabbits, rats, mice, fish, birds and the like. In some embodiments, the subject is a mammal. In some embodiments, the subject is a human. In some embodiments, the subject is a bird or fish. Thus, the methods may be carried out in the medical setting and the veterinary setting. The non-human animal subject may be, for example, a pet or an animal model of an ocular or non-ocular disease.

The phrase "topical administration" is used herein in its conventional sense to mean topical delivery to the desired anatomical site, such as the ocular surface. The fluid comprising high molecular weight hyaluronic acid may be applied directly or indirectly to the ocular surface by any manner that allows an effective amount of the fluid and ocular surface to make contact. For example, the fluid may be applied directly to the ocular surface, such as via eye drops or lavage, or applied indirectly via a delivery agent (*i.e.,* a fluid delivery agent) that is brought into contact with the ocular surface or other part of the eye. An example of a delivery agent is a particle (*e.g*., microparticles or nanoparticles) that is coated with the fluid and/or releases the fluid onto the ocular surface. Such particles may be composed of various materials, such as natural or synthetic polymers. In some embodiments, the delivery agent may itself be administered as drops.

The terms "treat", "treating" and "treatment" include alleviating, ameliorating, inhibiting or preventing the progress of, reversing or abrogating a medical condition, such as corneal nerve damage or loss, or one or more symptoms or complications associated with the condition, and alleviating, ameliorating or eradicating one or more causes of the condition.

As used herein, the expression "method of treating" and/or "method of preventing" is to be interpreted as a fluid of the invention for use in a method of treating or preventing.

The invention is described only exemplarily by the embodiments in the description and drawings and is not limited thereto but rather includes all variations, modifications, substitutions, and combinations the expert may take from the complete documents of this application under consideration of and/or combination with his specific knowledge.

Following are examples that illustrate procedures for practicing the invention. These examples should not be construed as limiting. All percentages are by weight and all solvent mixture proportions are by volume unless otherwise noted.

### Example 1 - High molecular weight hyaluronic acid eye drops promote corneal nerve growth

### A. Background

The HYLAN M study is a randomized clinical study on patients with severe dry eyes (according to the ODISSEY primary criteria), which is being conducted. Within the HYLAN M study, the patients are randomized in two groups, one staying with the most effective individual patient treatment identified before, the other one switched to high molecular weight hyaluronic acid eye drops (COMFORT SHIELD^{®} preservative-free sodium hyaluronate eye drops (i.com medical GmbH, Munich, Germany)), which corresponds to the embodiment of Table 1 herein.

These patients have already received the best treatment their ophthalmologists could offer. All of the patients had been under "stable" therapy at the time of their inclusion into the study, *i.e.,* their therapy has not been changed over a defined period of time prior to inclusion into this study. The patients are randomized into two groups, one group of patients remaining with their current therapy for dry eye syndrome, and the second group of patients treated with drops of the fluid described above (COMFORT SHIELD^{®} eye drops) in place of their tear substitute.

Study objectives include: (1) comparison of objective and subjective symptoms of dry eye under treatment with COMFORT SHIELD^{®} eye drops versus the tear substitute eye drops which the patients has been treated with before presenting to the investigator (=current therapy) in severe dry eye conditions; and (2) to observe objective performance, patients' subjective acceptance and adverse events of the eye drops. For each patient, both eyes are examined, and the eye with higher corneal fluorescein staining score at baseline examination is evaluated.

Within the HYLAN M Study, confocal microscopy is an optional diagnostic method to be performed at the baseline visit and the 8 weeks visit to analyze the sub-basal epithelial nerve plexus. Details concerning the HYLAN M Study follow.

Further details concerning the HYLAN M study can be found in MÜLLER-LIERHEIM, W.G.K. "The HYLAN M Study. Study design and first results" Akt. Kontaktol, January 2017, Issue 13, No. 27 (3 pages).

### B. General introduction to corneal confocal microscopy

The Heidelberg Retina Tomograph (HRT) is a confocal laser scanning system for the acquisition and analysis of two- or three-dimensional images of the posterior and anterior segment of the eye. The most important routine clinical application of the Heidelberg Retina Tomograph is the detection of glaucomatous damage of the optic nerve head and the follow-up of glaucomatous progression with the Glaucoma Module. The instrument enables the quantitative description of the optic nerve head topography and time related changes.

With the addition of the Rostock Cornea Module (RCM), the HRT is converted to a confocal corneal microscope that allows the acquisition of two- and three-dimensional images of the different corneal layers (including the corneal nerve plexus), as well as the limbus and conjunctiva.

For the acquisition of corneal images, a laser beam is focused on the cornea and periodically deflected by oscillating mirrors, so that a two-dimensional sector of the cornea is scanned sequentially. The amount of reflected light at each point is measured by a light-sensitive detector. In the confocal optical system of the HRT, light can only reach the detector if it is reflected or scattered from a narrow region surrounding the preset focal plane. Light reflected or scattered outside of the focal plane is highly suppressed. For this reason, a two-dimensional confocal image may be regarded as an enface optical section through the cornea. The focal plane can be moved manually through the entire cornea. Therefore, images of the following different corneal layers can be acquired.

The actual location of the focal plane is measured and stored together with each acquired image.

The laser source in the Heidelberg Retina Tomograph / Rostock Cornea Module is a diode laser with a wavelength of 670 microns. A two-dimensional image consists of 384 x 384 picture elements (pixels). It covers an area of 0.4 mm by 0.4 mm of the cornea using the "400 FOV" field lens, or an area of 0.3 mm by 0.3 mm of the cornea using the "300 FOV" field lens.

The device is designed to be operated by professional users with health care background and with experience in the operation of ophthalmic imaging and diagnostic equipment, such as physicians, ophthalmic photographers or optometrists.

### C. Corneal nerve plexus assessment

RCM measurements follow the clinical routine. Topical anesthetics and artificial tears are applied to both eyes of the subject, who is placed in the devices head- and chin-rest. The operator applies viscous artificial tears to the concave front part of the HRT-RCM patient interface and mount the "Tomocap" as contact element between HRT-RCM device and patients eye. The operator will then carefully move the HRT-RCM forward, until gentle contact is made between the "Tomocap" and the patient's cornea. The focal plane will be adjusted to the sub-basal nerve plexus layer and the images of the sub-basal nerve plexus should be collected in various locations close to the center of each cornea with special emphasis in the region of interest. Sub-basal nerve plexus images should be homogeneous illuminated, artifact free and have a maximal signal to noise ratio.

### D. Data collection within the HYLAN M study

10 sub-basal nerve plexus images of the central cornea region were collected for both eyes of each patient at the time of baseline examination and 8 weeks visit. The images were stored in single image mode of the HRT + RCM device and exported to a reading center for evaluation.

### E. Data analysis and Results

Images from 16 patients were collected at baseline and after 8 weeks treatment. These 16 patients included 8 patients of the control group who continued to use the control lubricant eye drops which they had been using by the time of inclusion in the HYLAN M study, and 8 patients for which the ocular lubricant eye drops had been substituted by COMFORT SHIELD^{®} eye drops.

To the inventors' surprise, a statistically significant (p = 0.031) growth of the total nerve fiber length (51% growth) over a period of 8 weeks in the patients treated with COMFORT SHIELD^{®} eye drops was found as compared to the control group, as shown in Figure 1. This neurotrophic activity is an unexpected effect.

### Example 2 - Detailed Analysis of the HYLAN M Study

As described in Example 1, patients suffering from severe dry eye disease (DED) were randomized into two parallel arms. The control group continued with their currently-used therapy by the time of inclusion. In the verum group (COMFORT SHIELD^{®} eye drops group; also referred to herein as the HMWHA group), the individual lubricant eye drops used by each patient by the time of inclusion were replaced by eye drops containing 0.15% HMWHA (COMFORT SHIELD^{®} eye drops, i.com medical GmbH, Munich, Germany). Concomitant treatment for dry eye, like cyclosporine eye drops, remained unchanged in both arms.

Demographic data and medical history were recorded during the baseline visits. Symptoms and signs associated with DED were assessed at the baseline visit, at week 4, and week 8 follow-up visits, respectively (see Table 2).

**Table 2. Diagnostic testing schedule with optional tests in round brackets.**

| **Test** | **Baseline** | **Week 4** | **Week 8** |
|---|---|---|---|
| OSDI | X | X | X |
| Dropping frequency | X | X | X |
| BCVA | X | X | X |
| CFS | X | X | X |
| TBUT | X | X | X |
| Schirmer 1 | X | | X |
| Tear osmolarity | X | | X |
| IOP | X | | X |
| LWE, Korb score (†) | (X) | | (X) |
| Yamaguchi score (††) | (X) | | (X) |
| Confocal microscopy | (X) | | (X) |

| | | | |
|---|---|---|---|
| Abbreviations: Ocular Surface Disease Index (OSDI), best corrected visual acuity (BCVA), corneal fluorescein staining (CFS), tear film break-up time (TBUT), intraocular pressure (IOP), and lid wiper epitheliopathy (LWE). †Korb, D.R. et al. Prevalence of lid wiper epitheliopathy in subjects with dry eye signs and symptoms. Cornea 2010, 29, 377-383. ††Yamaguchi, M. et al. Marx line: Fluorescein staining line on the inner lid as indicator of meibomian gland function. Am. J. Ophthalmol. 2006, 141, 669-675. | | | |

The study centers were suggested to optionally take CSLM images at the baseline and week eight visits and provide them to a masked reading center for assessment. Four out of 11 study centers participated in this optional test. These four study centers provided CSLM images of all their per-protocol patients; thus, the electronic randomization used throughout the HYLAN M study also applied to the optional confocal microscopy study. The results of the assessment of the CSLM images of these four study centers are the subject of this report. The results of the other diagnostic tests performed, such as the Ocular Surface Disease Index (OSDI), dropping frequency, best corrected visual acuity (BCVA), corneal fluorescein staining (CFS), tear film break-up time (TBUT), Schirmer 1, tear osmolarity, intraocular pressure (IOP), lid wiper epitheliopathy (LWE), and Yamaguchi score of all 84 per-protocol patients included in the HYLAN M study have been previously reported (van Setten et al., "The HYLAN M Study: Efficacy of 0.15% High Molecular Weight Hyaluronan Fluid in the Treatment of Severe Dry Eye Disease in a Multicenter Randomized Trial", J. Clin. Med. 2020, 9, 3536).

Patients over 18 years suffering from DED of any underlying etiology were eligible for inclusion. The patients had to be under stable, unchanged, dry eye therapy for at least two months (in case of concomitant cyclosporine therapy, three months) by the time of inclusion. Patients were excluded if they participated in any other clinical trial, suffered from eye diseases other than dry eyes, had ocular surgery less than three months prior to study inclusion, were using punctual plugs, or had masquerading conditions as identified by Karpecki (Karpecki, "Why dry eye trials often fail: From disease variability to confounding underlying conditions, there are countless reasons why new dry eye drugs have come up short in FDA testing," Rev. Optom. 2013, 150, 50-56). Masquerading conditions are conjunctivochalasis, recurrent corneal erosions, epithelial basement membrane dystrophy, mucus fishing syndrome, floppy eyelid syndrome, giant papillary conjunctivitis, Salzmann's nodular degeneration, and ocular rosacea.

As inclusion criteria for severe dry eye, the primary criteria, according to Baudouin *et al*., were chosen (Baudouin, C. et al. ODISSEY European Consensus Group Members. Diagnosing the severity of dry eye: A clear and practical algorithm. Br. J. Ophthalmol. 2014, 98, 1168-1176). The dry eye symptoms were assessed using the Ocular Surface Disease Index (OSDI) questionnaire, with an OSDI score of 33 or more being required for inclusion (Schiffman, R.M. et al. Reliability and validity of the Ocular Surface Disease Index. Arch. Ophthalmol. 2000, 118, 615-621). Corneal fluorescein staining (CFS) was selected as a dry eye sign (Bron, A.J. et al. Grading of corneal and conjunctival staining in the context of other dry eye tests. Cornea 2003, 22, 640-650). For inclusion, patients had to have at least one eye with CFS Oxford grade 3 or more, but no confluent CFS. The eyes with the higher staining score were defined as study eyes.

The Heidelberg Retina Tomograph (HRT 3), in combination with the Rostock Cornea Module (Heidelberg Engineering GmbH, Heidelberg, Germany), was used for the in vivo assessment of the corneal subbasal nerve plexus (SNP), as described previously (Ziegler, D. et al. Early detection of nerve fiber loss by corneal confocal microscopy and skin biopsy in recently diagnosed type 2 diabetes. Diabetes 2014, 63, 2454-2463; Stachs, O. et al. In Vivo Confocal Scanning Laser Microscopy. In High Resolution Imaging in Microscopy and Ophthalmology: New Frontiers in Biomedical Optics; Bille, J.F., Ed.; Springer: Cham, Switzerland, 2019; pp. 263-284). Both eyes were anesthetized with topical anesthetic and covered with artificial tears. To prevent eye movements, the patients were asked to fixate on a spotlight with the unexamined eye.

Five non-overlapping images were taken in the central region of the cornea, close to the apex and more than 0.5 mm apart from the inferior whorl (see Figure 2A for an example of an image and Figure 2B after image processing by the reading center).

Image processing and quantitative image analysis were performed by the reading center using Mathematica (Version 11.3, Wolfram Research Inc., Champaign, IL, USA), as previously described (Winter, K. et al. Local Variability of Parameters for Characterization of the Corneal Subbasal Nerve Plexus. Curr. Eye Res. 2016, 41, 186-198). The following SNP parameters were calculated: corneal nerve fiber length (CNFL), defined as the total length of all nerve fibers per unit area (mm/mm²); corneal nerve fiber density (CNFD), defined as the number of nerve fibers per unit area (n/mm²); corneal nerve branch density (CNBD), defined as the number of branching points per unit area (n/mm²); average weighted corneal nerve fiber tortuosity (CNFTo), reflected variability of nerve fiber directions and defined as absolute nerve fiber curvature/nerve fiber length (µm⁻¹); corneal nerve connection points (CNCP), defined as the number of nerve fibers crossing the area boundary (connections/mm²); average corneal nerve single-fiber length (CNSFL), defined as the average length of nerve fibers (µm); and average weighted corneal nerve fiber thickness (CNFTh), measured as mean thickness perpendicular to the nerve fiber course (µm).

Statistical analysis was performed using IBM SPSS Statistics (Version 22, IBM Corp., Armonk, New York, USA). Descriptive statistics were calculated, and box plots were generated. Data were examined for normal distribution using the Shapiro-Wilk test. Group comparisons were performed using the Wilcoxon Signed Rank Test and the Mann-Whitney U test, respectively. The significance level was determined to be *p* < 0.05.

Table 3 contains the socio-demographic characteristics of the patients with the CSLM assessment of the SNP.

**Table 3. Socio-demographic characteristics according to the treatment arm (n = 16).**

| | | **Comfort Shield *n =* 8** | **Control *n* = 8** |
|---|---|---|---|
| Age (years) | n | 8 | 8 |
| | mean (SD) | 59.5 (9.2) | 61.6 (18.4) |
| | min, max | 36, 77 | 47, 73 |
| Sex n (%) | n | 8 | 8 |
| | female | 6 (75) | 6 (75) |
| | male | 2 (25) | 2 (25) |
| Medical History | n | 8 | 8 |
| | Sjögren syndrome | 2 | 3 |

| | | **Comfort Shield *n =* 8** | **Control *n =* 8** |
|---|---|---|---|
| | rheumatoid disease | 3 | 2 |
| | rheumatoid + thyroid disease | 1 | |
| | thyroid disease | | 1 |
| | Graves disease + betablocker | 1 | |
| | diabetes mellitus + betablocker | | 1 |
| | no dry eye related disease | 1 | 1 |

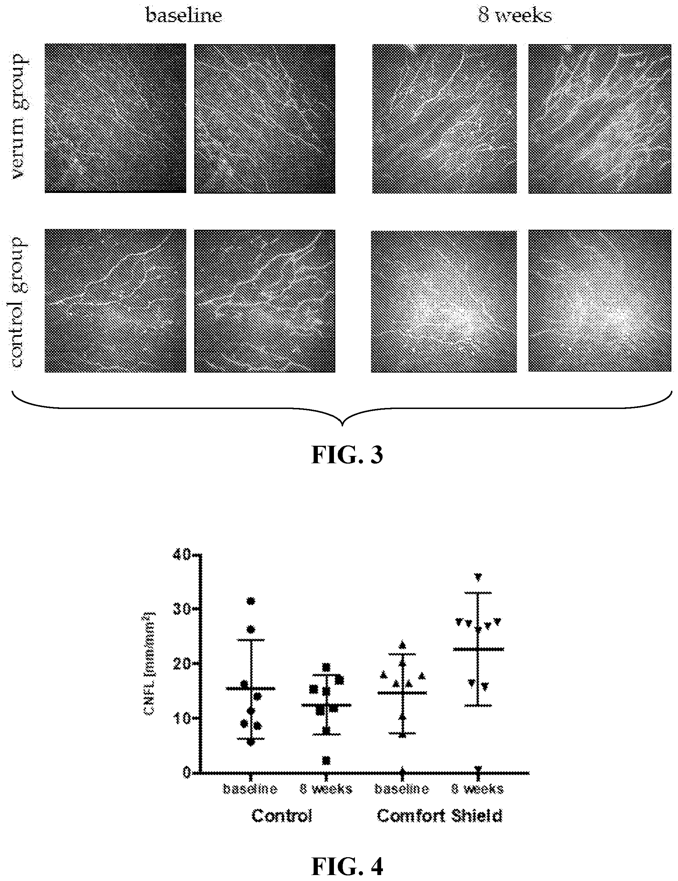

Five CSLM images of eight patients of the control group and eight patients of the HMWHA group taken at the end of the baseline visit and at the end of the week 8 visit were analyzed (see examples in Figure 3).

We found a statistically significant difference in CNFL between baseline and the eight weeks follow-up visit; the HMWHA group showed a significant difference in CNFL (*p =* 0.030) contrary to the control group (p = 0.294). CNFL was comparable for HMWHA and control at baseline (*p* = 0.793) and showed a significant difference after eight weeks (*p =* 0.031). Possibly due to the small number of patients, we did not find significant differences for the other SNP parameters (CNFD, CNBD, CNFTo, CNCP, CNSFL, CNFTh). Moreover, patients suffering from severe dry eye generally do not have a well-developed SNP, and there was a large amount of foreign tissue in the vicinity of the SNP that complicated the image analysis. Figure 4 summarized the CNFL findings of the HMWHA group and the control group at baseline and eight weeks visit. Figure 4 corresponds to Figure 1 in Example 1, with the inclusion of individual patient data points shown.

Further details concerning the HYLAN M study and the effects of COMFORT SHIELD^{®} eye drops on total nerve fiber length and the trophic effect at the subbasal nerve plexus are provided in van Setten et al. "The HYLAN M Study: Efficacy of 0.15% High Molecular Weight Hyaluronan Fluid in the Treatment of Severe Dry Eye Disease in a Multicenter Randomized Trial," J Clin Med., 2020 Nov 2; 9(11):3536; and van Setten et al., "High Molecular Weight Hyaluronan Promotes Corneal Nerve Growth in Severe Dry Eyes," J Clin Med., 2020 Nov 24; 9(12):3799.

## Claims

1. A fluid comprising high molecular weight hyaluronic acid (HMWHA) for use in treating or preventing corneal nerve damage or loss in a human or non-human animal subject, wherein the fluid is for topical administration to the ocular surface of the subject, wherein the hyaluronic acid has an intrinsic viscosity of at least 2.5 m³/kg, wherein the hyaluronic acid has a molecular weight in the range of 3 million to 4 million Daltons, and wherein the subject has diabetes and diabetic peripheral neuropathy, diabetes but does not yet have diabetic peripheral neuropathy, diabetic corneal neuropathy, stroke, neurotrophic keratopathy (NK), or corneal nerve damage or loss caused by trauma or a medical procedure.

2. The fluid for use according to claim 1, wherein the nerve damage or loss includes diminished nerve fiber length and/or diminished nerve fiber tortuosity.

3. The fluid for use according to claim 1, wherein the corneal nerve damage or loss in the subject was identified by *in vivo* confocal microscopy (IVCM) on the eye of the subject.

4. The fluid for use according to claim 1 or 2, wherein the subject has a sign or symptom of corneal nerve damage or loss, and preferably,
wherein the sign of corneal nerve damage or loss is one or more of the following: a decrease of corneal innervation or sensation, a reduction in the number of nerve fibers or bundles innervating the cornea, death of neurons innervating the cornea, a decrease or loss of neurotransmitter release, a decrease or loss of nerve growth factor release, abnormal tearing reflexes, abnormal blink reflexes, abnormal nerve morphology, appearance of abnormal nerve sprouts, abnormal tortuosity, increased bead-like nerve formations, thinning of nerve fiber bundles, thickening of nerve fiber bundles, diminished length of the inferior corneal nerve whorl, diminished density of corneal nerve, diminished length of corneal nerve, diminished branching of corneal nerve, recurrent corneal erosion, delayed epithelial wound healing of the cornea, or decreased tearing rate; or
wherein the symptom of corneal nerve damage or loss is one or more of the following: abnormal tear production or dryness, abnormal blinking, and difficulty or loss of ability to focus, decreased or lost visual acuity, or decreased or lost corneal sensitivity.

5. The fluid for use according to claim 1 or 2, wherein the subject has mild, moderate, or severe neurotrophic keratopathy (NK); or wherein the NK is mild (stage 1) NK or moderate (stage 2) NK at the time of administration, and the HMWHA fluid prevent or delays the progression of the NK to a state of severe (stage 3) NK.

6. The fluid for use according to claim 1 or 2, wherein the subject has type 1, type 2, or gestational diabetes and diabetic peripheral neuropathy; type 1, type 2, or gestational diabetes, but does not yet have diabetic peripheral neuropathy, or diabetic corneal neuropathy.

7. The fluid for use according to claim 1, wherein the trauma is chemical or mechanical trauma; or corneal nerve damage or loss is caused by a medical procedure.

8. The fluid for use according to claim 1 or 2, wherein the fluid is for administration in combination with one or more additional treatments before, during, or after topical administration of the HMWHA fluid, wherein the one or more additional treatments are selected from the group comprising: recombinant human nerve growth factor (cenegermin), matrix metalloproteinase inhibitor, plasma rich in growth factors (PRGF), therapeutic contact lens, temporary tarsorrhaphy (partial, or complete after HMWHA fluid is administered), amniotic membrane transplantation, penetrating keratoplasty, corneal transplantation, combination of cenegermin and corneal transplantation, or director or indirect corneal neurotization.

9. The fluid for use according to claim 1 or 2, wherein the HMWHA fluid is a drop or a wash, such as a lavage; or
wherein the HMWHA fluid is administered to the ocular surface by a fluid delivery agent that is for topical administration to the ocular surface, preferably wherein the delivery agent is a particle that is coated with and/or secretes the fluid on to the ocular surface.

10. The fluid for use according to claim 1 or 2, wherein the hyaluronic acid has an intrinsic viscosity in the range of 2.6 m³/kg to 2.9 m³/kg, or greater.

11. The fluid for use according to claim 1 or 2, wherein the HMWHA fluid is preservative-free.

12. The fluid for use according to claim 1 or 2, wherein the HMWHA fluid further comprises an additional glycosaminoglycan (GAG), an electrolyte such as sodium chloride, a buffer such as phosphate buffer, or a combination of two or more of the foregoing.

13. The fluid for use according to claim 1 or 2, wherein the HMWHA fluid comprises HMWHA having a concentration of < 0.2 % w/v, preferably, wherein the HMWHA fluid comprises HMWHA having a concentration of 0.1 to 0.19 % w/v, and more preferably, wherein the HMWHA fluid comprises HMWHA having a concentration of about 0.15 % w/v.

14. The fluid for use according to claim 1 or 2, wherein the HMWHA fluid has:
a) a pH of 6.8 - 7.6;
b) an osmolarity of 240 - 330 mOsmol/kg;
c) a NaCl concentration of 7.6 - 10.5 g/l; and/or
d) a phosphate concentration of 1.0 - 1.4 mmol/l.

## Patentansprüche

1. Fluid, umfassend Hyaluronsäure mit hohem Molekulargewicht (HMWHA) zur Verwendung bei der Behandlung oder Vorbeugung von Schäden oder von einem Verlust von Hornhautnerven in einem menschlichen oder nichtmenschlichen tierischen Subjekt, wobei das Fluid zur topischen Verabreichung an die Augenoberfläche des Subjekts vorgesehen ist, wobei die Hyaluronsäure eine intrinsische Viskosität von mindestens 2,5 m³/kg aufweist, wobei die Hyaluronsäure ein Molekulargewicht im Bereich von 3 Millionen bis 4 Millionen Dalton aufweist, und wobei das Subjekt Folgendes aufweist: Diabetes und diabetische periphere Neuropathie oder Diabetes, aber noch keine diabetische periphere Neuropathie, oder diabetische Hornhautneuropathie, Schlaganfall, neurotrophe Keratopathie (NK), Schäden oder Verlust von Hornhautnerven, verursacht durch ein Trauma oder einen medizinischen Eingriff.

2. Fluid zur Verwendung nach Anspruch 1, wobei die Schäden oder der Verlust eine verminderte Nervenfaserlänge und/oder eine verminderte Tortuosität der Nervenfasern einschließen/einschließt.

3. Fluid zur Verwendung nach Anspruch 1, wobei die Schäden oder der Verlust von Hornhautnerven bei dem Subjekt durch konfokale In-vivo-Mikroskopie (IVCM) am Auge des Subjekts identifiziert wurden/wurde.

4. Fluid zur Verwendung nach Anspruch 1 oder 2, wobei das Subjekt ein Anzeichen oder Symptom von Schäden oder Verlust von Hornhautnerven aufweist, und bevorzugt,
wobei das Anzeichen von Schäden oder eines Verlusts von Hornhautnerven eines oder mehrere der folgenden ist: eine Abnahme der Hornhautinnervation oder Hornhautsensibilität, eine Verringerung der Anzahl von Nervenfasern oder -bündeln, die die Hornhaut innervieren, Tod von Neuronen, die die Hornhaut innervieren, eine Abnahme oder ein Verlust der Neurotransmitterfreisetzung, eine Abnahme oder ein Verlust der Nervenwachstumsfaktorfreisetzung, anormale Tränenreflexe, anormale Blinkreflexe, anormale Nervenmorphologie, Auftreten anormaler Nervensprosse, anormale Tortuosität, vermehrte perlenartige Nervenformationen, Verdünnung von Nervenfaserbündeln, Verdickung von Nervenfaserbündeln, verminderte Länge des inferioren Hornhautnervenwirbels, verminderte Dichte von Hornhautnerven, verminderte Länge von Hornhautnerven, verminderte Verzweigung von Hornhautnerven, rezidivierende Hornhauterosion, verzögerte epitheliale Wundheilung der Hornhaut oder verringerte Tränenbildungsrate; oder
wobei das Symptom von Schäden oder eines Verlusts von Hornhautnerven eines oder mehrere der folgenden ist: anormale Tränenproduktion oder Trockenheit, anormales Blinzeln und Schwierigkeit oder Verlust der Fähigkeit, zu fokussieren, verringerte oder verlorene Sehschärfe oder verringerte oder verlorene Hornhautsensibilität.

5. Fluid zur Verwendung nach Anspruch 1 oder 2, wobei das Subjekt leichte, mäßige oder schwere neurotrophe Keratopathie (NK) aufweist; oder wobei die NK zum Zeitpunkt der Verabreichung leichte (Stadium 1) NK oder mäßige (Stadium 2) NK ist, und das HMWHA-Fluid das Fortschreiten der NK zu einem Zustand schwerer NK (Stadium 3) verhindert oder verzögert.

6. Fluid zur Verwendung nach Anspruch 1 oder 2, wobei das Subjekt Typ-1-, Typ-2- oder Gestationsdiabetes und diabetische periphere Neuropathie; Typ-1-, Typ-2- oder Gestationsdiabetes, aber noch keine diabetische periphere Neuropathie, oder diabetische Hornhautneuropathie aufweist.

7. Fluid zur Verwendung nach Anspruch 1, wobei das Trauma ein chemisches oder mechanisches Trauma ist; oder die Schäden oder der Verlust von Hornhautnerven durch einen medizinischen Eingriff verursacht sind/ist.

8. Fluid zur Verwendung nach Anspruch 1 oder 2, wobei das Fluid zur Verabreichung in Kombination mit einer oder mehreren zusätzlichen Behandlungen vor, während oder nach topischer Verabreichung des HMWHA-Fluids vorgesehen ist, wobei die eine oder die mehreren zusätzlichen Behandlungen aus der Gruppe ausgewählt sind, die Folgendes umfasst: rekombinanter humaner Nervenwachstumsfaktor (Cenegermin), Matrix-Metalloproteinase-Hemmer, wachstumsfaktorenreiches Plasma (PRGF), therapeutische Kontaktlinse, temporäre Tarsorrhaphie (partiell, oder vollständig, nachdem das HMWHA-Fluid verabreicht worden ist), Amnionmembrantransplantation, perforierende Keratoplastik, Hornhauttransplantation, Kombination aus Cenegermin und Hornhauttransplantation oder direkte oder indirekte korneale Neurotisation.

9. Fluid zur Verwendung nach Anspruch 1 oder 2, wobei das HMWHA-Fluid ein Tropfen oder eine Spülung ist, wie etwa eine Lavage; oder
wobei das HMWHA-Fluid an die Augenoberfläche durch ein Fluidverabreichungsmittel verabreicht wird, das zur topischen Verabreichung an die Augenoberfläche vorgesehen ist, bevorzugt wobei das Verabreichungsmittel ein Partikel ist, das mit dem Fluid beschichtet ist und/oder das Fluid auf die Augenoberfläche absondert.

10. Fluid zur Verwendung nach Anspruch 1 oder 2, wobei die Hyaluronsäure eine intrinsische Viskosität im Bereich von 2,6 m³/kg bis 2,9 m³/kg oder mehr aufweist.

11. Fluid zur Verwendung nach Anspruch 1 oder 2, wobei das HMWHA-Fluid konservierungsmittelfrei ist.

12. Fluid zur Verwendung nach Anspruch 1 oder 2, wobei das HMWHA-Fluid weiter ein zusätzliches Glykosaminoglykan (GAG), einen Elektrolyten wie Natriumchlorid, einen Puffer wie Phosphatpuffer oder eine Kombination aus zwei oder mehr der Vorstehenden umfasst.

13. Fluid zur Verwendung nach Anspruch 1 oder 2, wobei das HMWHA-Fluid HMWHA mit einer Konzentration von < 0,2 % w/v umfasst, bevorzugt wobei das HMWHA-Fluid HMWHA mit einer Konzentration von 0,1 bis 0,19 % w/v umfasst, und bevorzugter wobei das HMWHA-Fluid HMWHA mit einer Konzentration von etwa 0,15 % w/v umfasst.

14. Fluid zur Verwendung nach Anspruch 1 oder 2, wobei das HMWHA-Fluid Folgendes aufweist:
a) einen pH-Wert von 6,8 - 7,6;
b) eine Osmolarität von 240 - 330 mOsmol/kg;
c) eine NaCl-Konzentration von 7,6 - 10,5 g/l; und/oder
d) eine Phosphatkonzentration von 1,0 - 1,4 mmol/l.

## Revendications

1. Fluide comprenant de l'acide hyaluronique de haut poids moléculaire (HMWHA) pour utilisation dans le traitement ou la prévention d'une lésion ou perte de nerf cornéen chez un sujet animal non-humain ou humain, dans lequel le fluide est destiné à une administration topique à la surface oculaire du sujet, dans lequel l'acide hyaluronique présente une viscosité intrinsèque d'au moins 2,5 m³/kg, dans lequel l'acide hyaluronique présente un poids moléculaire dans la plage de 3 millions à 4 millions de Daltons, et dans lequel le sujet présente un diabète et une neuropathie périphérique diabétique, un diabète mais pas encore de neuropathie périphérique diabétique, une neuropathie cornéenne diabétique, un accident vasculaire cérébral, une kératopathie neurotrophique (NK) ou une lésion ou perte de nerf cornéen causée par un traumatisme ou une procédure médicale.

2. Fluide pour utilisation selon la revendication 1, dans lequel la lésion ou perte de nerf inclut une longueur de fibre nerveuse diminuée et/ou une sinuosité de fibre nerveuse diminuée.

3. Fluide pour utilisation selon la revendication 1, dans lequel la lésion ou perte de nerf cornéen chez le sujet a été identifiée par microscopie confocale in vivo (IVCM) sur l'œil du sujet.

4. Fluide pour utilisation selon la revendication 1 ou revendication 2, dans lequel le sujet présente un signe ou symptôme de lésion ou perte de nerf cornéen, et de préférence,
dans lequel le signe de lésion ou perte de nerf cornéen est un ou plusieurs des éléments suivants : une diminution de l'innervation ou de la sensibilité cornéenne, une réduction du nombre de fibres nerveuses ou de faisceaux innervant la cornée, une mort de neurones innervant la cornée, une diminution ou perte de libération de neurotransmetteurs, une diminution ou perte de libération de facteur de croissance nerveuse, des réflexes lacrymaux anormaux, des réflexes de clignement anormaux, une morphologie nerveuse anormale, une apparition de bourgeons nerveux anormaux, une sinuosité anormale, une augmentation de formations nerveuses en forme de perles, un amincissement de faisceaux de fibres nerveuses, un épaississement de faisceaux de fibres nerveuses, une longueur diminuée du vortex nerveux cornéen inférieur, une densité diminuée du nerf cornéen,
une longueur diminuée du nerf cornéen, une ramification diminuée du nerf cornéen, une érosion cornéenne récurrente, une cicatrisation retardée d'une plaie épithéliale de la cornée, ou un taux lacrymal diminué ; ou dans lequel le symptôme de lésion ou perte de nerf cornéen est un ou plusieurs des éléments suivants : une sécheresse ou production lacrymale anormale, un clignement anormal, et une difficulté ou perte de la capacité de concentration, une diminution ou perte de l'acuité visuelle, ou une diminution ou perte de la sensibilité cornéenne.

5. Fluide pour utilisation selon la revendication 1 ou revendication 2, dans lequel le sujet présente une kératopathie neurotrophique (NK) légère, modérée ou sévère ; ou dans lequel la NK est une NK légère (stade 1) ou une NK modérée (stade 2) au moment de l'administration, et le fluide HMWHA prévient ou retarde la progression de la NK vers un état de NK sévère (stade 3).

6. Fluide pour utilisation selon la revendication 1 ou revendication 2, dans lequel le sujet présente un diabète de type 1, de type 2 ou gestationnel et une neuropathie périphérique diabétique ; un diabète de type 1, de type 2 ou gestationnel, mais pas encore de neuropathie périphérique diabétique, ou une neuropathie cornéenne diabétique.

7. Fluide pour utilisation selon la revendication 1, dans lequel le traumatisme est un traumatisme chimique ou mécanique ; ou la lésion ou perte de nerf cornéen est causée par une procédure médicale.

8. Fluide pour utilisation selon la revendication 1 ou revendication 2, dans lequel le fluide est destiné à une administration en combinaison avec un ou plusieurs traitements supplémentaires avant, pendant ou après l'administration topique du fluide HMWHA, dans lequel les un ou plusieurs traitements supplémentaires sont sélectionnés dans le groupe comprenant : facteur de croissance nerveuse humain recombinant (cénégermine), inhibiteur de métalloprotéinase matricielle, plasma riche en facteurs de croissance (PRGF), lentille de contact thérapeutique, tarsorraphie temporaire (partielle, ou complète après administration du fluide HMWHA), greffe de membrane amniotique, kératoplastie transfixiante, greffe de cornée, combinaison de cénégermine et de greffe de cornée, ou neurotisation cornéenne directe ou indirecte.

9. Fluide pour utilisation selon la revendication 1 ou revendication 2, dans lequel le fluide HMWHA est une goutte ou un bain, tel qu'un lavage ; ou
dans lequel le fluide HMWHA est administré à la surface oculaire par un agent d'administration de fluide qui est destiné à une administration topique à la surface oculaire, de préférence dans lequel l'agent d'administration est une particule qui est revêtue du fluide et/ou sécrète le fluide à la surface oculaire.

10. Fluide pour utilisation selon la revendication 1 ou revendication 2, dans lequel l'acide hyaluronique présente une viscosité intrinsèque dans la plage de 2,6 m³/kg à 2,9 m³/kg, ou plus.

11. Fluide pour utilisation selon la revendication 1 ou revendication 2, dans lequel le fluide HMWHA est sans conservateur.

12. Fluide pour utilisation selon la revendication 1 ou revendication 2, dans lequel le fluide HMWHA comprend en outre un glycosaminoglycane (GAG) supplémentaire, un électrolyte tel que le chlorure de sodium, un tampon tel qu'un tampon phosphate, ou une combinaison de deux des éléments qui précèdent ou plus.

13. Fluide pour utilisation selon la revendication 1 ou revendication 2, dans lequel le fluide HMWHA comprend du HMWHA présentant une concentration de < 0,2 % p/v, de préférence, dans lequel le fluide HMWHA comprend du HMWHA présentant une concentration de 0,1 à 0,19 % p/v, et plus préférablement, dans lequel le fluide HMWHA comprend du HMWHA présentant une concentration d'environ 0,15 % p/v.

14. Fluide pour utilisation selon la revendication 1 ou revendication 2, dans lequel le fluide HMWHA présente :
a) un pH de 6,8 à 7,6 ;
b) une osmolarité de 240 à 330 mOsmol/kg ;
c) une concentration en NaCl de 7,6 à 10,5 g/l ; et/ou
d) une concentration en phosphate de 1,0 à 1,4 mmol/l.
